# EUROPEAN PATENT APPLICATION

(11) **EP 4 616 821 A1**
(43) Date of publication of application: **17.09.2025**
(21) Application number: 24305381.6
(22) Date of filing: 13.03.2024
(51) Int. Cl.: A61B 34/30, A61B 46/10, A61B 17/00, A61B 90/00

(54) **STERILE INTERFACE FOR A ROBOTIC ARM**

(71) Applicant: MinMaxMedical, 38400 Saint-Martin-d'Hères (FR)
(72) Inventor: FOURNIER, Elie, 38400 SAINT-MARTIN-D'HERES (FR); OUDOIRE, Patrick, 38400 SAINT-MARTIN-D'HERES (FR); COLLET, Hervé, 38400 SAINT-MARTIN-D'HERES (FR)
(74) Representative: Regimbeau

(57) **Abstract**

The present invention relates to a sterile interface (200) configured to interface an end-effector (107) of a robotic arm and a surgical instrument, the end-effector comprising a motor (109) configured to rotate the surgical instrument, the sterile interface comprising:
a sterile ring (210) configured to be detachably attached to the end-effector (107) of the robotic arm,
an instrument adaptor (220) configured to be detachably attached to the end-effector (107) of the robotic arm, the instrument adaptor comprising at least one ball bearing (225) configured to allow rotation of a shaft of the motor around a first axis (X),
the sterile ring and the end-effector being configured to cooperate to maintain a sterile drape between the sterile ring and the end-effector,
the sterile ring being further configured to prevent displacements of the instrument adaptor along the first axis (X).

## Description

The present invention relates to a sterile interface for a robotic arm. The present invention further relates to a surgical system including the sterile interface.

Operating rooms are subjected to very strict rules to ensure that the surgeon(s) and their assistants are working in sterile conditions. Over the last few decades, the use of surgical robots has become more and more common. Obviously, these surgical robots also have to comply with the sterility rules.

Surgical robots generally comprise a robotic arm made of several segments linked to one another by motorized joints, which holds a surgical tool adapted to treat an anatomical region of interest of a patient. This surgical tool can be a passive tool, such as a guide, or it can be an active tool, such as an oscillating surgical saw or a drill, and thus be activated by a motor also integrated to the robotic arm. During the surgery, a sterile zone is defined surrounding the patient, and particularly around the anatomical region of interest of said patient. Any object entering the defined sterile zone must be sterilized. As they contain electronic pieces, the motorized joints as well as the optional surgical tool motor cannot be easily sterilized and thus, have to be covered with a sterile drape during the surgery to prevent contaminations. This sterile drape thus forms a sterile barrier between the robot and the sterile zone.

Obviously, the surgical tool must enter the sterile zone to treat the anatomical region of interest and thus such surgical tool must be sterilized beforehand. Thus, there is a need to provide a piece permitting to connect the surgical tool to the motor and/or to parts of the robotic arm which are under the sterile drape, while ensuring that the sterile barrier is preserved. Also, this assembly should be easy, quick and secure. Moreover, it is preferable to provide a piece which can be mounted on the surgical system by a single person as human resources in operating rooms are often limited.

The present invention falls in this context by proposing a sterile interface configured to interface an end-effector of a robotic arm and a surgical instrument, the end-effector comprising a motor configured to rotate the surgical instrument, the sterile interface comprising:
a sterile ring configured to be detachably attached to the end-effector of the robotic arm,
an instrument adaptor configured to be detachably attached to the end-effector of the robotic arm, the instrument adaptor comprising at least one ball bearing configured to allow rotation of a shaft of the motor around a first axis,
the sterile ring and the end-effector being configured to cooperate to maintain a sterile drape between the sterile ring and the end-effector,
the sterile ring being further configured to prevent displacements of the instrument adaptor along the first axis.

The invention may be complemented by one or several of the following features, alone or in combination.

The sterile ring comprises at least one locking member configured to engage with the instrument adaptor to lock a position of the sterile ring with respect to the instrument adaptor.

The locking member comprises at least one flexible tab configured to engage with at least one notch formed on the instrument adaptor.

The sterile ring comprises at least one abutment surface configured to abut against the instrument adaptor, so as to prevent the displacements of the instrument adaptor along the first axis.

The abutment surface is formed on an internal face of the sterile ring.

The instrument adaptor extends, at least partially, through the sterile ring.

The sterile ring is configured to be attached to the end-effector with a bayonet connection.

The sterile ring is configured to be screwed to the end-effector.

The sterile ring comprises a collar extending radially with respect to the first axis, the collar being configured to cooperate with the end-effector of the robotic arm so as to maintain the sterile drape between the sterile ring and the end-effector.

The instrument adaptor comprises a connecting part configured to be detachably attached to the end-effector, the connecting part being configured to cooperate with the flange of the robotic arm so as to prevent rotation of the instrument adaptor around the first axis.

The connecting part comprises at least one tooth configured to cooperate with a corresponding slot of the flange of the robotic arm.

The connecting part of the instrument adaptor is configured to provide a poka-yoke to the assembly of the instrument adaptor with the flange.

The connecting part of the instrument adaptor is configured to guide the assembly of the instrument adaptor with the flange.

Another object of the present invention concerns a surgical system comprising a cart, a robotic arm, an end-effector, a sterile interface as mentioned above, a sterile drape maintained between the sterile ring of the sterile interface and the end-effector and a surgical instrument, the robotic arm extending from the cart to a flange, the end-effector being detachably attached to the flange of the robotic arm and the sterile interface interfacing the end-effector and the surgical instrument.

The sterile drape can comprise a through-hole, the instrument adaptor and the sterile ring extending at least partially through the through-hole.

The present invention further proposes a method for assembling a sterile interface as mentioned above, comprising the steps of
attaching the instrument adaptor on the end-effector;
threading the sterile drape onto the instrument adaptor and the end-effector;
threading the sterile ring onto the instrument adaptor to sandwich the sterile drape with the end-effector;
manually engaging the locking member of the sterile ring with the instrument adaptor.

At least the steps of attaching the instrument adaptor and of threading the sterile drape can be performed in any order, or even be implemented simultaneously within the scope of the invention.

Other features and advantages of the invention will appear in the following description with reference to the drawings, in which
- FIG. 1 is a general overview of a surgical system equipped with a sterile interface of the invention;
- FIG. 2 is a perspective view of the sterile interface of the invention mounted on an end-effector of a robotic arm of the surgical system illustrated on FIG. 1;
- FIG. 3 and 4 are perspective views of a sterile ring of the sterile interface of the invention, respectively a front view and a rear view of the sterile ring;
- FIG. 5 is a perspective view of an instrument adaptor of the sterile interface of the invention;
- FIG. 6A shows the sterile interface viewed from a surgical instrument of the surgical system;
- FIG. 6B is a longitudinal cross-section view of the sterile interface according to a plan A-A illustrated on figure 6A;
- FIG. 7A and 7b are perspective views illustrating an assembly method of the sterile interface according to an embodiment of the invention.

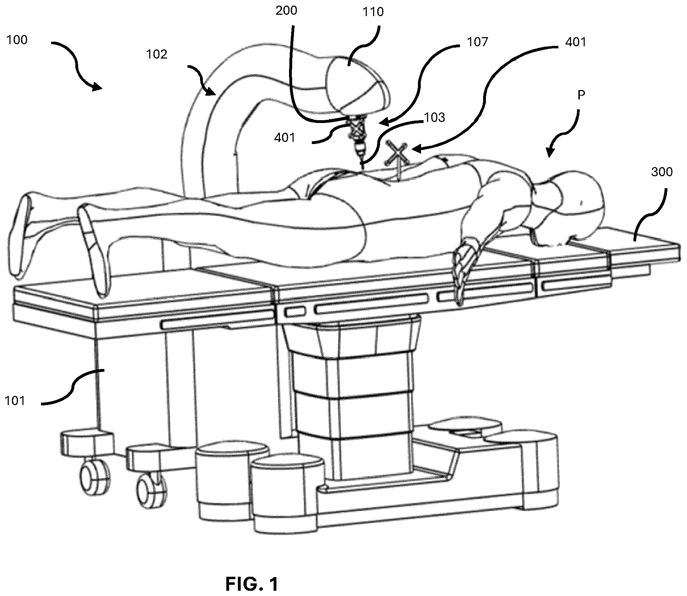

FIG. 1 illustrates a general overview of a surgical system 100 which comprises a sterile interface 200 of the invention and a sterile drape 110. This sterile drape 110 forms a sterile barrier defining a sterile zone - outside of the sterile drape - and a non-sterile zone - inside of said sterile drape. As shown, a patient P is installed on an operating table 300 in the sterile zone.

The surgical system 100 comprises a cart 101 from which extends a robotic arm 102 holding a surgical instrument 103. The robotic arm 102 comprises several segments linked to one another by motorized joints. The segments and motorized joints are not visible on figure 1, as hidden by the sterile drape 110. Alternatively, the joints could be deprived of motor without departing from the scope of the present invention.

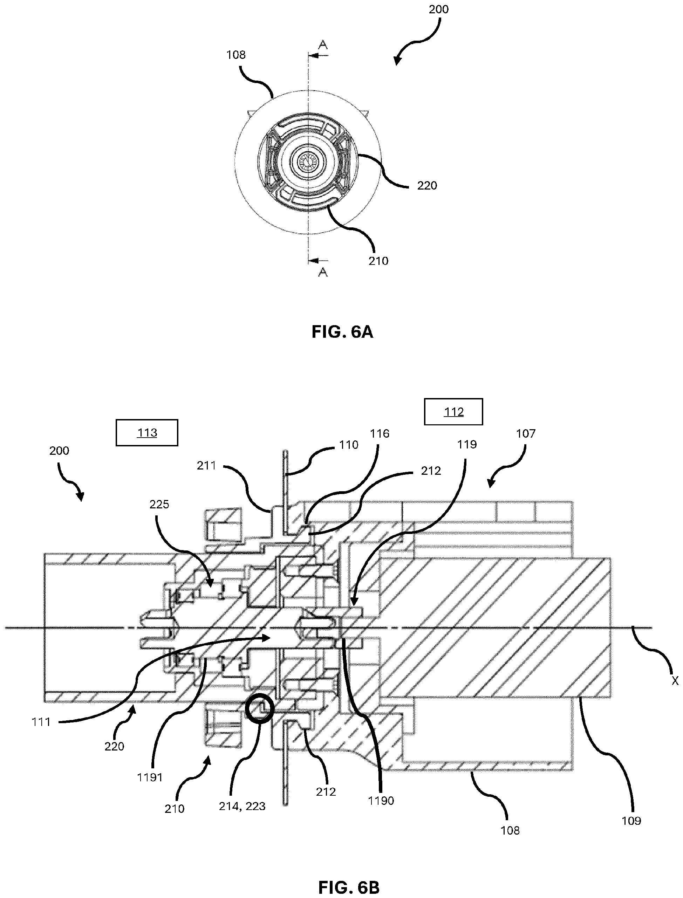

The last segment of the robotic arm 102 ends with a flange to which an end-effector 107 is attached. Advantageously, the end-effector 107 can be detachably attached to the flange. The end-effector 107 comprises a housing 108 and a motor housed in said housing 108 - as shown on figure 6B. The surgical instrument 103 is more particularly detachably attached to this end-effector 107, and as detailed below, the motor of the end-effector 107 is configured to rotate a motor shaft, itself configured to rotate, directly or indirectly the surgical instrument 103. As well known in the art, a converter can optionally be interposed between the motor and the surgical instrument, for instance for converting a rotation of the motor shaft into an oscillation when the surgical instrument is an oscillating saw.

As shown, the surgical system 100 can also optionally comprise a navigation system configured to determine the relative pose of, at least, the surgical instrument 103 held by the robotic arm with respect to the patient P, and particularly with respect to a region of interest of the patient, which is to be treated. According to the illustrated embodiment, the navigation system is an optical tracking system comprising optical trackers 401 and a stereoscopic camera - not shown here. A first optical tracker 401 is attached to the end-effector 107 and a second optical tracker 401 is attached to the patient P. Obviously, this is only an example, and the navigation system could comprise an electromagnetic tracking system and/or an inertial measurement device.

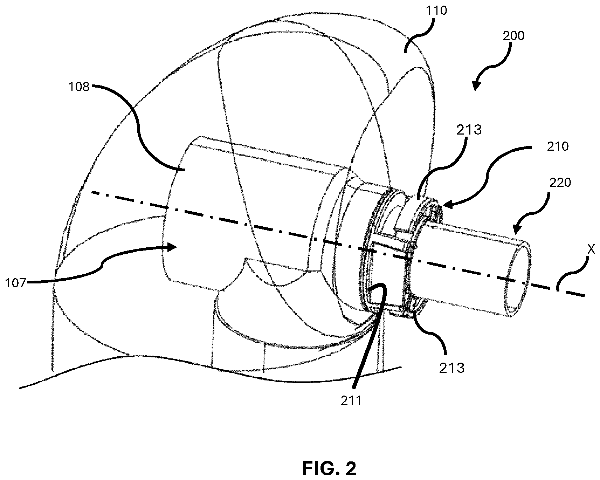

FIG. 2 is a perspective view of the sterile interface 200 mounted on the end-effector 107 of the robotic arm. The sterile interface 200 comprises a sterile ring 210 and an instrument adaptor 220. The end-effector 107 may comprise a fixation plate which can for instance be configured to allow fixation of an electronic casing of the end-effector - not shown here.

As illustrated, the instrument adaptor 220 extends along a first axis X, and at least partially through the sterile ring 210. The sterile drape 110 is sandwiched between the end-effector 107 and the sterile ring 210, thus forming the sterile barrier. Particularly, the sterile drape 110 is maintained between the end-effector 107 and a collar 211 of the sterile ring 210.

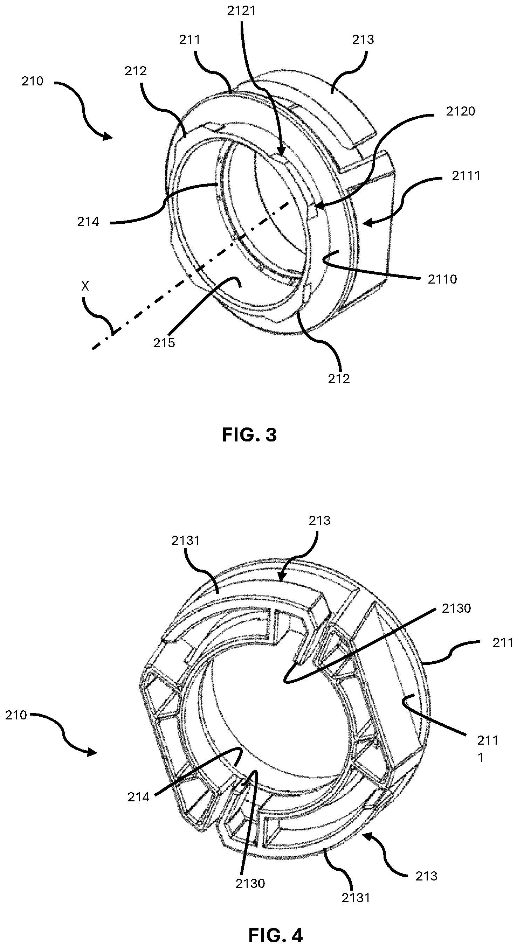

Perspective views of the sterile ring 210, respectively a front view and a rear view, are illustrated on figures 3 and 4. As mentioned, the sterile ring 210 comprises a collar 211 configured to cooperate with the end-effector 107 of the robotic arm to maintain the sterile drape in position, thus forming the sterile barrier. This collar 211 extends radially with respect to the first axis X, from all a circumference of the sterile ring 210. According to the illustrated embodiment, the collar is made of a single piece with the sterile ring 210. Thus, the collar 211 cannot be separated from the sterile ring 210 without damaging at least one of the collar or the sterile ring.

The sterile ring 210 is detachably attached to the end-effector 107 and especially to the housing 108 of this end-effector. For instance, the sterile ring can be screwed to the end-effector. Alternatively, the sterile ring can be detachably attached to the end-effector with a bayonet connection, or the sterile ring can be clipped on the end-effector. According to the illustrated embodiment, the sterile ring 210 is screwed by a quarter turn to the end-effector. To do so, at least one wing 212 is arranged on the sterile ring 210. According to the illustrated embodiment, the sterile ring 210 comprises four wings 212 configured to cooperate with a corresponding groove 116 arranged in the end-effector - as for instance illustrated on FIG. 6B. Each wing 212 is shaped as a ramp, having a first end 2120 thicker than its second end 2121. Obviously, the corresponding groove 116 presents a complementary shape.

In order to allow a user of the robotic arm to engage - and disengage - the sterile ring 210 with the end-effector, such sterile ring 210 comprises at least one locking member 213. This locking member 213 formed on the sterile ring 210 is configured to engage with the instrument adaptor to lock the position of the sterile ring 210 with respect to the instrument adaptor and to prevent unwanted disengagement. The locking member is particularly configured to prevent disengagement of the sterile ring 210 from the instrument adaptor when the surgical instrument is powered and generating vibrations.

Advantageously, this locking member 213 can further be provided with a safety mechanism allowing the user of the robotic system to ensure that the sterile ring 210 is locked on the instrument adaptor.

According to an embodiment, this locking member comprises at least one flexible tab 213 configured to engage with at least one notch formed in the instrument adaptor. Obviously, the instrument adaptor presents at least as many notches as the sterile ring comprises flexible tabs.

According to the illustrated embodiment, the locking member comprises two flexible tabs 213 arranged on opposed sides of the sterile ring 210. By "opposed sides" we here mean that these flexible tabs are diametrically opposed. Each flexible tab 213 presents a rigid portion 2130 configured to engage with one of the notches formed on the instrument adaptor and a flexible portion 2131. The engagement of the rigid portion 2130 with the corresponding notch locks the position of the sterile ring 210 with respect to the instrument adaptor and prevents unwanted disengagement. Advantageously, when the rigid portion 2130 engages with the corresponding notch, a "click" sound can be heard, informing the user of the system that the sterile ring 210 is securely engaged with the instrument adaptor. The flexible portion 2131 helps the user grasping the sterile ring to engage/disengage it on and off the instrument adaptor. Pressing the flexible portions 2131 further permits to lift the corresponding rigid portions 2130, thus facilitating the engagement of the sterile ring on the instrument adaptor and permitting the disengagement of said rigid portions 2130 from the notches, thus permitting the disengagement of the sterile ring.

As mentioned above, the sterile ring 210 comprises a collar 211 which extends radially with respect to the first axis, from an external circumference of the sterile ring 210. According to the illustrated embodiment, the collar 211 extends around the entirety of the external circumference of the sterile ring. Alternatively, this collar 211 could be formed only on part of said external circumference. The collar 211 presents two faces, a first face 2110 configured to face the end-effector of the robotic arm and a second face 2111 opposed to the first face 2110. When the sterile interface is mounted on the robotic arm, the sterile drape is thus sandwiched between the end-effector and the first face 2110 of the collar 211. Optionally, an O-ring can be added between the first face 2110 of the collar 211 and the sterile drape, in order to ensure that the sterile drape is correctly maintained.

The sterile ring 210 further comprises an abutment surface 214 configured to abut against the instrument adaptor and to prevent its displacement along the first axis X, as detailed below with reference to FIG. 6B. Particularly, the abutment surface 214 is formed on an internal face 215 of the sterile ring 210. According to the illustrated example, the abutment surface 214 extends on a complete circumference of the internal face 215 of the sterile ring 210. Alternatively, the abutment surface 214 could be formed only on a portion of the internal face of the sterile ring.

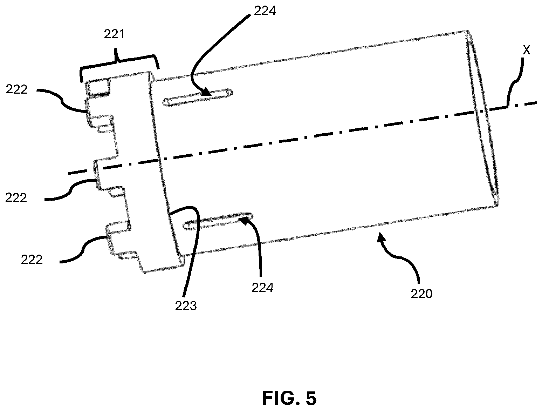

FIG. 5 illustrates, in a perspective view, the instrument adaptor 220. The instrument adaptor 220 has a general cylindrical shape extending along the first axis X. Obviously, the instrument adaptor 220 could present any other shape compatible with the invention. As detailed below with reference to FIG. 6B, the instrument adaptor 220 comprises a ball bearing configured to cooperate with a motor shaft of the motor housed in the end-effector of the robotic arm.

The instrument adaptor 220 is configured to be detachably attached to the end-effector of the robotic arm. The instrument adaptor 220 comprises at one of its ends, a connecting part 221 configured to cooperate with the end-effector of the robotic arm. This connecting part 221 is configured to lock a rotation of the instrument adaptor with respect to the end-effector, around the first axis X. Advantageously, this connecting part further permits to center the instrument adaptor 220 with respect to the end-effector.

The connecting part 221 comprises at least one tooth 222, advantageously several teeth 222 configured to cooperate with corresponding slot(s) arranged on the end-effector, as for instance illustrated on FIG. 6B. These teeth 222 thus form the connecting part 221 of the instrument adaptor 220. The connecting part 221 further forms a guiding part, configured to guide the insertion of the instrument adaptor 220, and particularly helps centering the instrument adaptor 220 with respect to the end-effector of the robotic arm.

As already mentioned, the instrument adaptor 220 is locked in translation along the first axis X thanks to the abutment surface arranged on the internal face of the sterile ring. As illustrated on FIG. 6, the abutment surface is more particularly configured to abut against a shoulder 223 of the connecting part 221.

FIG. 5 also illustrates the notches 224 described above and configured to engage with the rigid portions of the flexible tabs. Advantageously, the instrument adaptor 220 can present more notches 224 than the sterile ring comprises flexible tabs, thus permitting to engage the sterile ring according to several positions with respect to the instrument adaptor 220.

FIG. 6A shows the sterile interface 200 viewed from the surgical instrument. FIG. 6B is a longitudinal cross-section view of the sterile interface 200 taken along a longitudinal plane A-A illustrated on figure 6A. On FIG. 6B, the sterile interface 200 is illustrated together with the sterile drape 110. As mentioned above, this sterile drape 110 is sandwiched between the sterile ring 210 and the end-effector 107 of the robotic arm, and more particularly between the collar 211 of the sterile ring 210 and said end-effector 107.

The sterile ring 210 is further configured to prevent displacement of the instrument adaptor 220 along the first axis X thanks to its abutment surface 214 which abuts against the shoulder 223 of the connecting part 221 of the instrument adaptor 220. Advantageously, a sealing device, such as an O-ring for instance, can be interposed between the sterile ring 210 and the instrument adaptor 220 to ensure a tight connection, thus ensuring the efficiency of the sterile barrier. Particularly, the sealing device - not illustrated here - can be interposed between the abutment surface 214 and the shoulder 223. This sealing device also forms a compression device, participating to secure the engagement of the sterile ring 210 around the instrument adaptor 220. This compression device further permits to compensate for manufacturing tolerances. Optionally, such a compression device could be formed as a spring, or any other known device, arranged between the abutment surface 214 and the shoulder 223 to ensure the compression.

FIG. 6B also illustrates the cooperation between the wings 212 of the sterile ring 210 and the corresponding groove 116 arranged in the end-effector 107. Particularly, this groove 116 is arranged on an internal surface of the housing 108 of the end-effector 107. This cooperation between the wings 212 and the groove 116 permits to attach the sterile ring 210 on the end-effector 107, and thus participates to the maintaining of the sterile drape 110 in position, sandwiched between the collar 211 of the sterile ring 210 and the end-effector 107.

FIG. 6B further illustrates the cooperation of the ball bearing 225 of the instrument adaptor 220 with the motor shaft 119 of the motor 109 housed in the end-effector 107 of the robotic arm. Particularly, the motor shaft 119 is made of at least two parts, a first part 1190 being rotated by the motor and a second part 1191 cooperating with the ball bearing 225, thus transmitting the rotation of the motor 109 to the surgical instrument. According to the illustrated example, the first part 1190 of the motor shaft 119 is configured to transmit the rotational movement to its second part 1191 via a V-tooth coupling comprising at least two teeth. Such a configuration is only an example and can be replaced by any other equivalent configuration as well-known by the skilled person of the art.

This cross-section view of FIG. 6B finally shows that a through-hole 111 is formed in the sterile drape 110. As illustrated, at least part of the instrument adaptor 220 and part of the sterile ring 210 extend through this through-hole 111. Using a pierced sterile drape 110 permits to transmit mechanical force from the motor 109 arranged in the non-sterile zone 112, to the surgical instrument which is operated in the sterile zone 113.

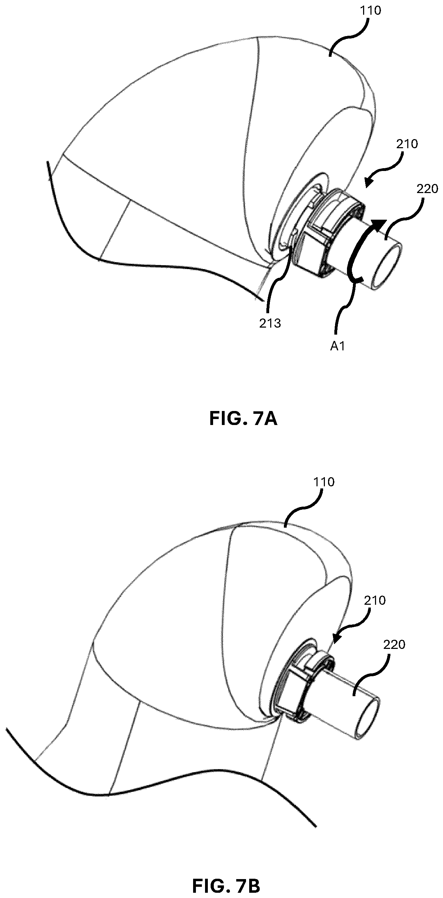

A method for assembling the sterile interface 200 of the invention is schematically illustrated on FIG. 7A and 7B and comprises the steps of
attaching the instrument adaptor 220 on the end-effector;
threading the sterile drape 110 onto the instrument adaptor 220 and the end-effector;
threading the sterile ring 210 onto the instrument adaptor 220 to sandwich the sterile drape 110 with the end-effector;
manually engaging the locking member 213 of the sterile ring with the instrument adaptor 220.

At least the steps of attaching the instrument adaptor 220 and of threading the sterile drape 110 can be performed in any order, or even be implemented simultaneously within the scope of the invention.

According to the illustrated embodiment, the manual engagement of the locking member of the sterile ring 210 comprise a translation of said sterile ring 210 along a direction parallel to the first axis X combined to a rotation of said sterile ring 210 along the arrow A1 to engage wings with the corresponding groove and to engage the locking member 213 with the instrument adaptor. As mentioned above, the sterile ring 210 can alternatively be clipped to the end-effector. FIG. 7B illustrates the sterile interface of the invention, once all the steps of the assembly method are completed.

Advantageously, the instrument adaptor can be attached to the end-effector before entering the operating room. Consequently, the nurse in charge of preparing such operating room does not need any tool to prepare the surgical system of the invention. It is thus understood from the foregoing that the sterile interface 200 of the invention provides a quick and easy assembly, particularly as such assembly does not require any tool once in the operating room. Moreover, the sterile interface of the invention is provided with element guiding the assembly, such as the connecting part, thus ensuring a correct assembly with only a few minutes of learning for the person in charge of this assembly.

## Claims

1. Sterile interface (200) configured to interface an end-effector (107) of a robotic arm (102) and a surgical instrument (103), the end-effector comprising a motor (109) configured to rotate the surgical instrument, the sterile interface comprising:
a sterile ring (210) configured to be detachably attached to the end-effector (107) of the robotic arm,
an instrument adaptor (220) configured to be detachably attached to the end-effector (107) of the robotic arm, the instrument adaptor comprising at least one ball bearing (225) configured to allow rotation of a shaft of the motor around a first axis (X),
the sterile ring and the end-effector being configured to cooperate to maintain a sterile drape between the sterile ring and the end-effector,
the sterile ring being further configured to prevent displacements of the instrument adaptor along the first axis (X).

2. Sterile interface according to the preceding claim, wherein the sterile ring (210) comprises at least one locking member (213) configured to engage with the instrument adaptor (220) to lock a position of the sterile ring with respect to the instrument adaptor.

3. Sterile interface according to the preceding claim, wherein the locking member (213) comprises at least one flexible tab configured to engage with at least one notch (224) formed on the instrument adaptor (220).

4. Sterile interface according to any of claims 1 to 3, wherein the sterile ring (210) comprises at least one abutment surface (214) configured to abut against the instrument adaptor (220), so as to prevent the displacements of the instrument adaptor along the first axis (X).

5. Sterile interface according to the preceding claim, wherein the abutment surface (214) is formed on an internal face (215) of the sterile ring (210).

6. Sterile interface according to any of claims 1 to 5, wherein the instrument adaptor (220) extends, at least partially, through the sterile ring (210).

7. Sterile interface according to any of claims 1 to 6, wherein the sterile ring (210) is configured to be attached to the end-effector (107) with a bayonet connection.

8. Sterile interface according to any of claims 1 to 6, wherein the sterile ring (210) is configured to be screwed to the end-effector (107).

9. Sterile interface according to any of the preceding claims, wherein the sterile ring (210) comprises a collar (211) extending radially with respect to the first axis (X), the collar being configured to cooperate with the end-effector (107) of the robotic arm so as to maintain the sterile drape between the sterile ring and the end-effector.

10. Sterile interface according to any of the preceding claims, wherein the instrument adaptor (220) comprises a connecting part (221) configured to be detachably attached to the end-effector (107), the connecting part being configured to cooperate with the end-effector (107) of the robotic arm (102) so as to prevent rotation of the instrument adaptor (220) around the first axis (X).

11. Sterile interface according to the preceding claim, wherein the connecting part (221) comprises at least one tooth (222) configured to cooperate with a corresponding slot of the end-effector (107) of the robotic arm (102).

12. Sterile interface according to any of claims 10 or 11, wherein the connecting part (221) of the instrument adaptor (220) is configured to provide a poka-yoke to the assembly of the instrument adaptor with the end-effector.

13. Sterile interface according to any of claims 10 to 12, wherein the connecting part (221) of the instrument adaptor (220) is configured to guide the assembly of the instrument adaptor with the end-effector.

14. Surgical system (100) comprising a cart (101), a robotic arm (102), an end-effector (107), a sterile interface (200) according to any of the preceding claims, a sterile drape (110) maintained between the sterile ring (210) of the sterile interface and the end-effector, and a surgical instrument (103), the robotic arm extending from the cart to a flange, the end-effector being detachably attached to the flange of the robotic arm and the sterile interface (200) interfacing the end-effector (107) and the surgical instrument (103).

15. Surgical system (100) according to the preceding claim, wherein the sterile drape comprises a through-hole (111), the instrument adaptor (220) and the sterile ring (210) extending at least partially through the through-hole.

16. Method for assembling a sterile interface (200) according to any of claims 1 to 13 in combination with claim 2, comprising:
attaching the instrument adaptor (220) on the end-effector (107);
threading the sterile drape (110) onto the instrument adaptor (220) and the end-effector (107);
threading the sterile ring (210) onto the instrument adaptor (220) to sandwich the sterile drape (110) with the end-effector (107);
manually engaging the locking member (213) of the sterile ring (210) with the instrument adaptor (220).
